(19) Europäisches Patentamt
European Patent Office
Office européen des brevets

(11) **EP 3 207 861 A2**

(12) **EUROPEAN PATENT APPLICATION**

(43) Date of publication:
**23.08.2017 Bulletin 2017/34**

(51) Int Cl.:
**A61B 3/113** *(2006.01)*

(21) Application number: **17155791.1**

(22) Date of filing: **13.02.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **22.02.2016   JP 2016031477**

(71) Applicant: **FUJITSU LIMITED**
**Kawasaki-shi Kanagawa 211-8588 (JP)**

(72) Inventor: **Nakashima, Satoshi**
**Kanagawa, 211-8588 (JP)**

(74) Representative: **Hoffmann Eitle**
**Patent- und Rechtsanwälte PartmbB**
**Arabellastraße 30**
**81925 München (DE)**

(54) **DETECTION SYSTEM AND DETECTION METHOD**

(57)     A detection system detects corneal reflection to be used to detect an eye gaze of a subject. The detection system includes a detection unit configured to detect reflected light from a subject side irradiated with light from a light source and generate an image based on a detection result of the reflected light, an extraction unit configured to extract a region from the image, the region having an intensity of the reflected light which is equal to or larger than a specific value, and a determination unit configured to, when there are two or more regions, as a extraction result of the region, in which a first region having a characteristic indicating movement over time coexist with a second region having another characteristic indicating no movement over time, determine the first region as the corneal reflection.

## FIG. 2

EP 3 207 861 A2

**Description**

FIELD

The embodiment discussed herein is related to a technique to detect an eye gaze of a subject.

BACKGROUND

There is a technique of a corneal reflection method using a near-infrared light source and a near-infrared camera to detect an eye gaze of a person that is a subject (for example, An Eye Tracking System Based on Eye Ball Model -Toward Realization of Gaze Controlled Input Device by Takehiko Ohno et al., the research report of Information Processing Society of Japan, 2001-HI-93, 2001, pp. 47-54). In the corneal reflection method, near-infrared light is reflected by a cornea (corneal reflection) by using the near-infrared light source, and a center position of the corneal reflection and a center position of the pupil are obtained by image processing. Then, in the corneal reflection method, an eye gaze of the subject is detected based on a positional relationship between the center position of the reflection and the center position of the pupil.

Moreover, when the subject whose eye gaze is to be detected is a person wearing eyeglasses, for example, near-infrared light emitted from the near-infrared light source is reflected on the lens surface of the eyeglass other than the corneal surface. Hereinafter, the reflection on the lens surface of the eyeglasses is referred to as eyeglass reflection. In the corneal reflection method, when the eyeglass reflection is detected as the corneal reflection, a wrong direction or position is detected as an eye-gaze direction or eye-gaze position.

Therefore, there have been proposed various eye-gaze detection techniques considering the eyeglass reflection (for example, Japanese Laid-open Patent Publication Nos. 2006-167256 and 2012-239550). For example, a certain corneal reflection determination device extracts an eye region from each frame of image data and detects corneal reflection candidates from the eye region in the image data. Then, the corneal reflection determination device finds, among the multiple corneal reflection candidates, a corneal reflection candidate extracted only intermittently in the frames of the image data, and determines the selected corneal reflection candidate as the corneal reflection. More specifically, the corneal reflection determination device excludes continuously extracted corneal reflection candidates as the eyeglass reflection by utilizing the fact that the corneal reflection disappears while the eyeglass reflection does not disappear in a frame of image data captured at the moment of a blink.

SUMMARY

TECHNICAL PROBLEM

As described above, in order to accurately detect the eye gaze, an attempt has been made to accurately detect the corneal reflection by excluding the eyeglass reflection. However, the corneal reflection determination device described above may fail in successfully excluding the eyeglass reflection in some cases depending on capabilities of the camera and other reasons. For example, if the camera fails to capture image data at the moment of a blink, it is impossible to exclude the eyeglass reflection. For this reason, it is inevitable for the camera to have a certain frame rate or higher.

Therefore, according to one aspect, it is an object of the technique disclosed herein to provide another method for detecting an eye gaze of a subject wearing eyeglasses.

SOLUTION TO PROBLEM

According to an aspect of the invention, a detection system detects corneal reflection to be used to detect an eye gaze of a subject. The detection system includes a detection unit configured to detect reflected light from a subject side irradiated with light from a light source and generate an image based on a detection result of the reflected light, an extraction unit configured to extract a region from the image, the region having an intensity of the reflected light which is equal to or larger than a specific value, and a determination unit configured to, when there are two or more regions, as a extraction result of the region, in which a first region having a characteristic indicating movement over time coexist with a second region having another characteristic indicating no movement over time, determine the first region as the corneal reflection.

BRIEF DESCRIPTION OF DRAWINGS

FIGs. 1A to 1C are diagrams for explaining a method for detecting an eye gaze according to this embodiment;
FIG. 2 is a diagram illustrating a configuration of an eye-gaze detection system according to this embodiment;
FIG. 3 is a functional block diagram illustrating functions of a controller;
FIG. 4 is a table illustrating a data configuration example of a storage unit configured to store corneal reflection candidate information;
FIGs. 5A and 5B are explanatory diagrams of circumferential pixels;
FIGs. 6A and 6B are diagrams (Part 1) illustrating a brightness gradient of corneal reflection candidates;

FIGs. 7A and 7B are diagrams (Part 2) illustrating a brightness gradient of corneal reflection candidates;
FIG. 8 is a table illustrating a data configuration example of a storage unit configured to store corneal reflection information;
FIG. 9 is a diagram for explaining tracking processing;
FIG. 10 is a flowchart (Part 1) illustrating eye-gaze detection processing;
FIG. 11 is a flowchart (Part 2) illustrating eye-gaze detection processing;
FIG. 12 is a flowchart (Part 3) illustrating eye-gaze detection processing;
FIG. 13 is a flowchart illustrating the tracking processing; and
FIG. 14 is a diagram illustrating a hardware configuration of the eye-gaze detection system including an eye-gaze detection device.

DESCRIPTION OF EMBODIMENT

Hereinafter, with reference to the drawings, detailed description is given of an embodiment of an eye-gaze detection technique. Note that the technique disclosed in the embodiment is not limited to the embodiment.

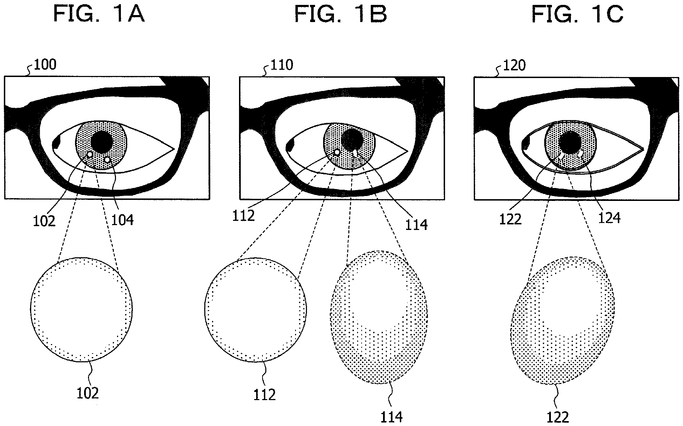

FIGs. 1A to 1C are diagrams for explaining a method for detecting an eye gaze according to this embodiment. FIG. 1A is a diagram illustrating a part 100 (right eye region) of an image taken in a state where the face of a subject for eye-gaze detection remains still as well as his/her eyeball. FIG. 1B is a diagram illustrating a part 110 of an image taken in a state where the eyeball of the subject is in motion while his/her face remains still. FIG. 1C is a diagram illustrating a part 120 of an image taken in a state where the face of the subject is in motion.
In the part 100 of the image illustrated in FIG. 1A, two reflections (102 and 104) are taken. These reflections (102 and 104) are on objects (eyeball and eyeglass) not in motion. Both the reflections (102 and 104) have a small difference in brightness between the vicinity of the outlines of the reflections (102 and 104) and the central portions thereof, and have larger difference in brightness with the surroundings (black part of the eye) of the reflections, than reflections (114, 122, and 124) to be described later. Therefore, both the reflections (102 and 104) are sharply defined. Note that FIG. 1A illustrates only an enlarged view of the reflection 102. However, the same goes for the reflection 104, and there is no particularly large difference in characteristics between both the reflections (102 and 104).
Next, two reflections (122 and 124) are taken similarly in a part 120 of an image illustrated in FIG. 1C. These reflections (122 and 124) are on objects (eyeball and eyeglass) in motion. As for both the reflections (122 and 124), the brightness gets gradually lower toward the outline from the vicinity of the centers of the reflections (122 and 124). Also, the brightness gradient within the region is gentler than in the reflections (102 and 104) described above, and a difference in brightness with the surroundings (black part of the eye) is smaller than in the reflections (102 and 104) described above. Therefore, both the reflections (122 and 124) are vaguely outlined. Note that FIG. 1C illustrates only an enlarged view of the reflection 122. However, the same goes for the reflection 124, and there is no particularly large difference in characteristics between both the reflections (122 and 124).
Here, in FIG. 1C, a situation that the corneal reflection as well as the eyeglass reflection are moved in the facial movement direction since the face is in motion is recorded in an exposure time of a camera. Therefore, the reflections (122 and 124) are gradually moved, and the movement locus is shot in a blurry state. Note that, in FIGs. 1A to 1C, white indicates the highest brightness and the finer hatching indicates lower brightness.
On the other hand, the part 110 of the image illustrated in FIG. 1B includes both the sharply outlined reflection 112 and the vaguely outlined reflection 114. In the state where the image illustrated in FIG. 1B is taken, the outline of the eyeglass reflection on the eyeglass surface does not get blurred, since the face including the eyeglass part is not in motion. However, since the eyeball is in motion, the corneal reflection is moved and shot in a blurry state on the image. This is because the movement of only the corneal reflection in the eyeball movement direction is recorded in the exposure time of the camera. Therefore, considering such characteristics, a distinction may be made that the reflection 112 is the eyeglass reflection and the reflection 114 is the corneal reflection.
Note that, as human characteristics, eye gaze movement and cassard phenomena frequently occur even without facial or head movement. Such eye gaze movement and cassard phenomena also occur when the face is in motion. However, when the face is in motion, the facial movement is larger and thus the influence of the facial movement becomes dominant in the state of FIG. 1C.
The inventors have focused attention on the existence of the images illustrated in FIGs. 1A to 1C that vary depending on the states where the images are taken, and particularly found out that the use of the image illustrated in FIG. 1B makes it possible to make a distinction between the eyeglass reflection and the corneal reflection. More specifically, in order to enable eye gaze detection by a corneal reflection method even if there is eyeglass reflection, the inventors have found a method of determining, when a corneal reflection candidate indicating reflection on an object in motion coexists with a corneal reflection candidate indicating reflection on an object not in motion, the former as the corneal reflection.
As described in detail below, in this embodiment, a detection device configured to detect a corneal reflection used to detect an eye gaze of a subject detects reflected light from the subject side irradiated with light from a light source, extracts a region where the reflected light has intensity of a predetermined value or higher, based on the detection result, and determines, when there are two or more regions in which a first region having a characteristic indicating movement over time coexists with a second region having a characteristic indicating no movement over time, the first region as a corneal reflection region representing the corneal reflection.

Note that, in FIGs. 1A to 1C, the presence or absence of movement is identified using the fact that the movement of the reflection in the exposure time is shot as blur. However, a method for identifying the presence or absence of movement is not limited thereto. For example, the presence or absence of movement may be identified based on images having a short exposure time.

As described above, in this embodiment, the eyeglass reflection and the corneal reflection may be distinguished from each other by using characteristic images even when there is eyeglass reflection, regardless of the capabilities of the camera. Therefore, the technique disclosed in this embodiment accurately detects the corneal reflection and reduces erroneous eye-gaze detection by using the detection result on the corneal reflection.

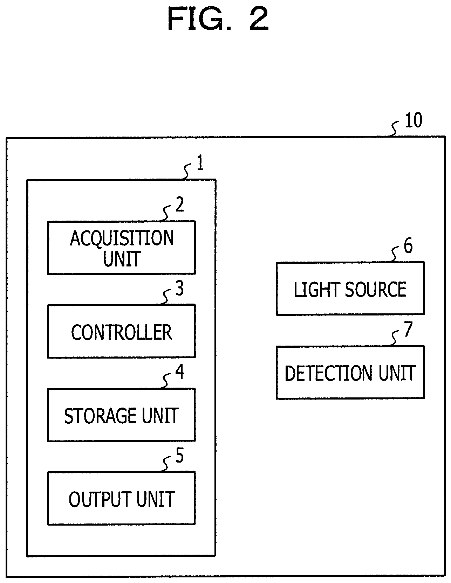

The embodiment is described in detail below. FIG. 2 is a diagram illustrating a configuration of an eye-gaze detection system according to this embodiment. As illustrated in FIG. 2, an eye-gaze detection system 10 includes an eye-gaze detection device 1, a light source 6, and a detection unit 7. The light source 6 irradiates a subject with light having a predetermined wavelength.

The detection unit 7 detects light reflected from the subject side irradiated with the light from the light source 6. The detection unit 7 is, for example, a camera sensitive to the light having the predetermined wavelength, which is emitted by the light source 6. Thus, the detection unit 7 detects light reflected from the subject side irradiated with the light from the light source 6, by shooting the subject. Note that the subject is a person whose eye gaze is to be detected. Also, the subject side represents the subject and other objects (including eyeglasses).

In this embodiment, near-infrared light invisible to the subject is used as the light having the predetermined wavelength. Thus, the camera as an example of the detection unit 7 is a near-infrared light camera. The light source 6 is an LED (Light Emitting Diode) that emits near-infrared light. An image to be acquired as the detection result by the detection unit 7 is a near-infrared image. The near-infrared image captures the situation of the subject side at brightness levels corresponding to the intensities of the reflection of the near-infrared light emitted by the light source 6 and of reflection of near-infrared light (for example, natural light or fluorescent light) emitted from another light source.

The eye-gaze detection device 1 detects an eye gaze of the subject. Note that the eye-gaze detection device 1 is, for example, a computer including a processor configured to execute various kinds of processing and a memory configured to store information.

In the eye-gaze detection system 10, the light source 6 and the detection unit 7 are connected to the eye-gaze detection device 1. Note, however, that such connection may be realized by wireless communication, other than wired connection. For example, when eye-gaze detection processing is started, the detection unit 7 takes images of the subject side at regular time intervals under the control of the eye-gaze detection device 1, and outputs the images taken to the eye-gaze detection device 1. Also, when the eye-gaze detection processing is started, the eye-gaze detection device 1 controls lighting of the light source 6.

In this embodiment, the eye-gaze detection device 1 detects the eye gaze of the subject by executing the eye-gaze detection processing on a near-infrared image acquired from the detection unit 7. For example, the eye-gaze detection device 1 uses the near-infrared image to detect a center position of corneal reflection of the light emitted from the light source 6 and a center position of the pupil of the subject, thereby detecting the eye gaze using a corneal reflection method. Note that, for processing other than the detection of the corneal reflection according to this embodiment, a conventional technique of the corneal reflection method is basically used. For example, the method described in Ohno et al. is adopted.

Here, the result of the processing by the eye-gaze detection device 1 is used for marketing analysis, for example. To be more specific, when the detection unit 7 and the light source 6 are installed in a shelf or a plate of a product in a store, the eye-gaze detection device 1 detects an eye gaze of a customer (subject) who comes into the store. Then, in the marketing analysis, which product the customer is interested in is estimated from the result of the eye gaze detection, for example. Moreover, a product displayed in a position where many eye gazes are focused is specified by statistically processing the result of the eye-gaze detection. Thus, a product in which many customers are interested may be figured out based on the output (eye-gaze detection result) from the eye-gaze detection device 1.

Moreover, the processing result obtained by the eye-gaze detection device 1 is used to detect dangerous driving, for example. To be more specific, when the detection unit 7 is installed in a position where images of the eyes of a driver seated in a driver seat may be taken and the light source 6 is installed in a position where the eyes of the driver may be irradiated with light, the eye-gaze detection device 1 detects an eye gaze of the driver (subject). In detection of dangerous driving, whether or not the driver performs proper driving while paying attention in various directions, whether or not there is a risk of drowsy driving, and the like are estimated from the result of the eye gaze detection,.

As illustrated in FIG. 2, the eye-gaze detection device 1 includes an acquisition unit 2, a controller 3, a storage unit 4, and an output unit 5. The acquisition unit 2 sequentially acquires detection results from the detection unit 7, and inputs the acquired detection results to the controller 3. For example, the acquisition unit 2 sequentially acquires image information on near-infrared images taken of the face of the subject from the detection unit 7. The image information includes brightness information indicating the intensity of reflection of near-infrared light in each pixel. Note that the acquisition unit 2 functions as a receiver when the detection unit 7 and the eye-gaze detection device 1 communicate with each other through wireless communication.

The controller 3 detects the corneal reflection and the pupil from the detection result inputted by the acquisition unit 2, and controls eye-gaze detection processing to detect the eye gaze based on a center position of the corneal reflection and a center position of the pupil. Note that, in detection of the cornea, the controller 3 uses an image in which a corneal reflection candidate having a characteristic indicating movement over time coexists with a corneal reflection candidate having a characteristic indicating no movement over time. As for the corneal reflection candidate, the intensity of the reflected light in the detection result is a predetermined value or higher. The corneal reflection candidate is, for example, a region where pixels having brightness of 200 or more are assembled in a near-infrared image (in the case of 8-bit, the maximum value of brightness is 255). The details thereof are described later.

The storage unit 4 stores various information desired for the eye-gaze detection processing according to this embodiment. The storage unit 4 stores, for example, the detection result (image information) from the detection unit 7, which is acquired by the acquisition unit 2, information on the corneal reflection candidates, and the like. The details thereof are described later.

The output unit 5 outputs the detected eye-gaze information, as the eye-gaze detection result, to another device. Such another device may be a device configured to perform the marketing analysis described above, a device configured to detect the dangerous driving described above, or a display device such as a display. Note that the output unit 5 functions as a transmitter when the output unit 5 and another device communicate with each other through wireless communication.

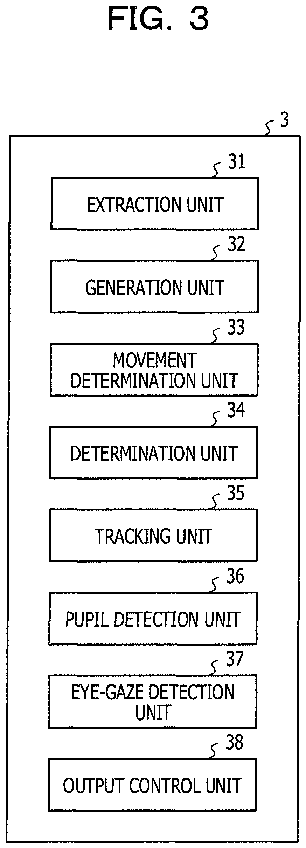

Next, the controller 3 is described in detail. FIG. 3 is a functional block diagram illustrating functions of the controller. The controller 3 includes an extraction unit 31, a generation unit 32, a movement determination unit 33, a determination unit 34, a tracking unit 35, a pupil detection unit 36, an eye-gaze detection unit 37, and an output control unit 38. Note that the extraction unit 31, the generation unit 32, the movement determination unit 33, the determination unit 34, and the tracking unit 35 in the controller 3 may also function as a corneal reflection detection device.

The extraction unit 31 extracts an approximately circular region where the reflection intensity (for example, brightness) is a predetermined value or higher, as a corneal reflection candidate, based on the detection result from the detection unit 7.

For example, the extraction unit 31 detects a face region from the acquired image, and further detects an eye region from the face region. Note that a conventional facial recognition technique is used for facial recognition and eye recognition. In the facial recognition technique, a template obtained by learning characteristics of a face and parts of the face is scanned on a process target image. Thus, a region having characteristics similar to the template is recognized as the face or parts of the face.

Next, the extraction unit 31 generates a binarized image by binarizing the eye region. Note that, in generation of the binarized image, the extraction unit 31 gives "1" to a pixel having brightness of 200 or more and "0" to a pixel having brightness smaller than 200, for example. Then, the extraction unit 31 further groups regions where a predetermined number of pixels or more with "1" given thereto are assembled. Note that such grouping processing is also called labeling processing.

Next, the extraction unit 31 determines whether or not the shape of each of the grouped regions is approximately circular. For such approximately circular shape determination, the technique disclosed in "Digital Image Processing", pp. 224-225, by Wilhelm Burger et al. or "Analysis of Snowflake Shape by a Region and Contour Approach" by Kenichiro Muramoto et al., The Journal of the Institute of Electronics, Information and Communication Engineers, May 1993, Vol. J76-D-II, No. 5, pp. 949-958, for example, may be used. For example, the extraction unit 31 evaluates the circularity among the characteristics of each region, and determines that the region is approximately circular when the circularity is a threshold or more.

Then, the extraction unit 31 stores the region determined to be approximately circular as the corneal reflection candidate in the storage unit 4. For example, the extraction unit 31 stores positional information on corneal reflection candidates and identification information on each of the corneal reflection candidates in the storage unit 4. Note that the positional information on the corneal reflection candidates is coordinates of the barycenter position and pixels on the circumference. Furthermore, the extraction unit 31 may extract image information of the regions stored as the corneal reflection candidates from image information (not the binarized images), and store the extracted image information.

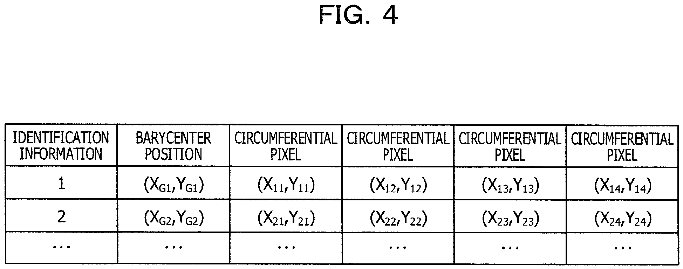

FIG. 4 is a table illustrating a data configuration example of the storage unit configured to store corneal reflection candidate information. The storage unit 4 stores, as the corneal reflection candidate information, identification information for identifying corneal reflection candidates and coordinates of a barycenter position and four circumferential pixels in association with each other. For example, when m corneal reflection candidates are extracted, 1 to m integers are given to the respective corneal reflection candidates as the identification information.

Moreover, in FIG. 4, the description is given of an example where four pixels above, below, to the right, and to the left of the barycenter are used as the circumferential pixels, for example. However, the positions and number of the circumferential pixels are not limited thereto.

For example, as for a region of a corneal reflection candidate having the identification "1" given thereto, the barycenter position is $(X_{G1}, Y_{G1})$ and coordinates of circumferential pixels are $(X_{11}, Y_{11})$, $(X_{12}, Y_{12})$, $(X_{13}, Y_{13})$, and $(X_{14}, Y_{14})$, respectively. Note that FIG. 4 presents the result of the corneal reflection candidate extraction processing on the image illustrated in FIG. 1B.

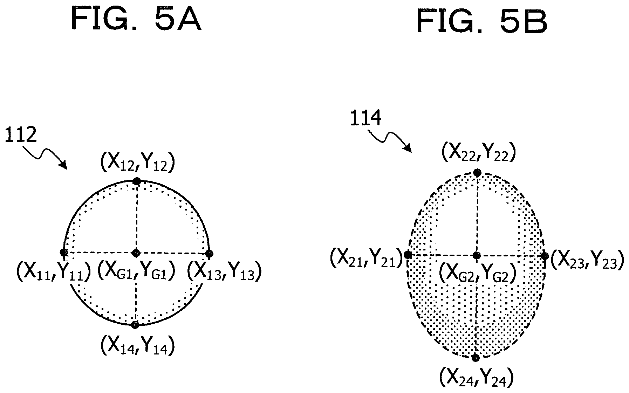

FIGs. 5A and 5B are explanatory diagrams of circumferential pixels. FIG. 5A illustrates an enlarged view of the reflection 112 in FIG. 1B as well as the circumferential pixels and the barycenter position in the reflection 112. FIG. 5A illustrates the barycenter position $(X_{G1}, Y_{G1})$ and the circumferential pixels $(X_{11}, Y_{11})$, $(X_{12}, Y_{12})$, $(X_{13}, Y_{13})$, and $(X_{14}, Y_{14})$ above, below, to the right, and to the left of the barycenter position. FIG. 5B illustrates an enlarged view of the reflection 114 in FIG. 1B as well as the circumferential pixels and the barycenter position in the reflection 114. FIG. 5B illustrates the barycenter position $(X_{G2}, Y_{G2})$ and the circumferential pixels $(X_{21}, Y_{21})$, $(X_{22}, Y_{22})$, $(X_{23}, Y_{23})$, and $(X_{24}, Y_{24})$ above, below, to the right, and to the left of the barycenter position.

Referring back to FIG. 3, the generation unit 32 generates characteristic information on the respective corneal reflection candidates. The characteristic information is information used to determine the presence or absence of movement, and is information indicating characteristics that vary depending on the presence or absence of movement. For example, for a region of a corneal reflection candidate determined to be approximately circular, the generation unit 32 generates information indicating a difference in length between a long axis and a short axis of an ellipse and a brightness gradient of the corneal reflection candidate region. For example, if a corneal reflection candidate has a large difference in length between the long axis and the short axis, this represents that the ellipticity is high and the corneal reflection candidate has a characteristic of movement as illustrated in FIGs. 1A to 1C.

Hereinafter, description is given of an example where the generation unit 32 generates brightness gradients of the corneal reflection candidate region in two or more directions (x direction, y direction, and others). However, in generation of the characteristic information, the generation unit 32 uses image information (information before being binarized) acquired from the detection unit 7, rather than the binarized information.

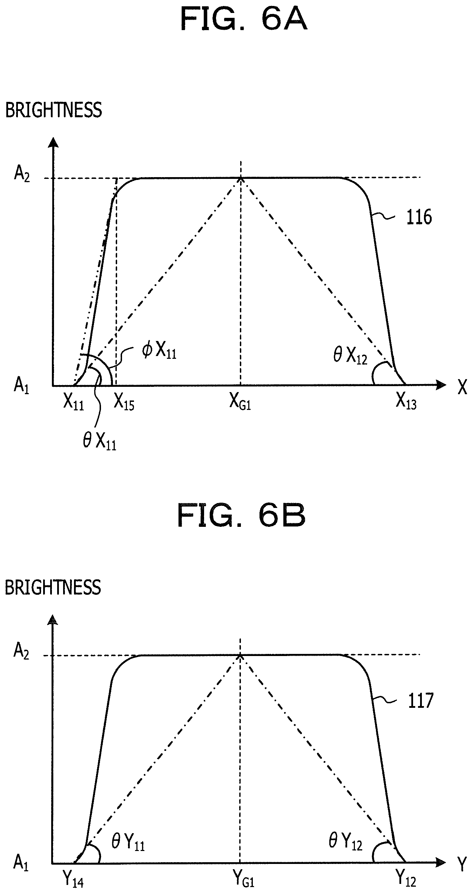

FIGs. 6A and 6B are diagrams (Part 1) illustrating the brightness gradients of the corneal reflection candidate. FIGs. 6A and 6B present the brightness gradients of the corneal reflection candidate 112. Also, here, description is given of an example where the brightness gradients are obtained in two directions, X direction and Y direction. However, the generation unit 32 may obtain brightness gradients in any number of directions, such as four directions and eight directions. Moreover, FIGs. 6A and 6B illustrate the brightness gradients as angles for convenience. However, the brightness gradient may be a slope itself, which is obtained by the following technique.

FIG. 6A illustrates a graph 116 (solid line) in which positions in the X direction and the brightness are plotted. The generation unit 32 obtains a brightness gradient $\theta_{X11}$ of the corneal reflection candidate 112 based on Equation 1 below. $A_1$ is the minimum value of the brightness of the corneal reflection candidate 112, while $A_2$ is the maximum value of the brightness of the corneal reflection candidate 112. Note that the brightness gradient $\theta_{X11}$ is equivalent to the slope of the dashed-dotted line in FIG. 6A.

$$\theta_{X11} = \frac{A_2 - A_1}{X_{G1} - X_{X11}} \qquad (1)$$

Furthermore, the generation unit 32 sets the brightness gradient $\theta_{X11}$ as a brightness gradient $\theta_{X1}$ in the X direction of the corneal reflection candidate 112. However, the generation unit 32 may also obtain left and right brightness gradients $(\theta_{X11}$ and $\theta_{X12})$ and sets the average thereof as the brightness gradient $\theta_{X1}$, in the X direction of the corneal reflection candidate 112. In this case, the generation unit 32 obtains the brightness gradient $\theta_{X12}$ based on Equation 1, obtains the average of $\theta_{X11}$ and $\theta_{X12}$, and sets the average as the brightness gradient $\theta_{X1}$ in the X direction of the corneal reflection candidate 112.

Moreover, a method for obtaining the brightness gradient is not limited to Equation 1. For example, the generation unit 32 may obtain a brightness gradient $\varphi_{X11}$ based on Equation 2 below. The brightness gradient $\varphi_{X11}$ is equivalent to the slope of the double-dashed dotted line in FIG. 6A.

$$\phi_{X11} = \frac{A_2 - A_1}{X_{15} - X_{X11}} \qquad (2)$$

Next, FIG. 6B illustrates a graph 117 (solid line) in which positions in the Y direction and the brightness are plotted. The generation unit 32 obtains a brightness gradient $\theta_{Y11}$ based on Equation 3. Then, the generation unit 32 sets the brightness gradient $\theta_{Y11}$ as a brightness gradient $\theta_{Y1}$ in the Y direction of the corneal reflection candidate 112. Note that the generation unit 32 may obtain a brightness gradient $\theta_{Y12}$ and set the average of $\theta_{Y11}$ and $\theta_{Y12}$ as the brightness gradient $\theta_{Y1}$ in the Y direction of the corneal reflection candidate 112 based

on Equation 3.

$$\theta_{Y11} = \frac{A_2 - A_1}{Y_{G1} - Y_{X14}} \quad (3)$$

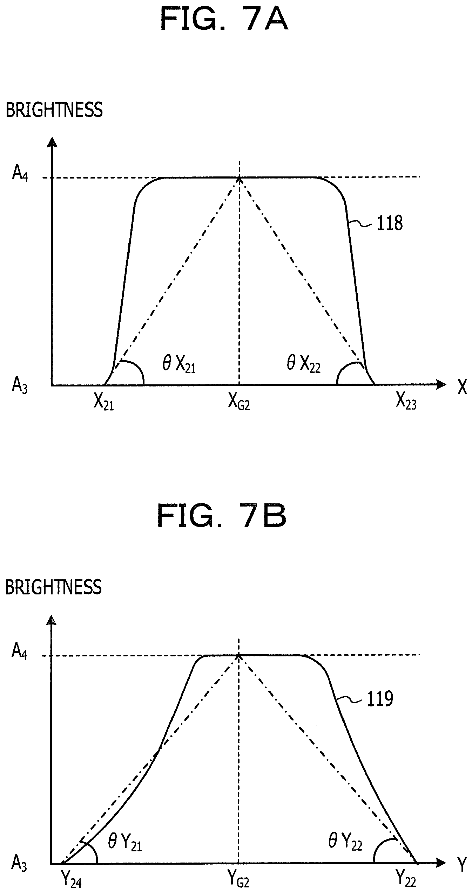

Next, FIGs. 7A and 7B are diagrams (Part 2) illustrating the brightness gradients of the corneal reflection candidate. FIGs. 7A and 7B present the brightness gradients of the corneal reflection candidate 114. FIG. 7A illustrates a graph 118 (solid line) in which positions in the X direction and the brightness are plotted. FIG. 7B illustrates a graph 119 (solid line) in which positions in the Y direction and the brightness are plotted.

The generation unit 31 obtains a brightness gradient $\theta_{X21}$ in the X direction in the same manner as Equation 1, and generates a brightness gradient $\theta_{X2}$ in the X direction of the corneal reflection candidate 114. Then, the generation unit 31 obtains a brightness gradient $\theta_{Y21}$ in the Y direction in the same manner as Equation 3, and generates a brightness gradient $\theta_{Y2}$ in the Y direction of the corneal reflection candidate 114. Note that, as in the case of FIGs. 6A and 6B, the generation unit 31 may obtain brightness gradients $\theta_{X22}$ and $\theta_{Y22}$ and then obtain $\theta_{X2}$ and $\theta_{Y2}$ from the averages thereof.

Referring back to FIG. 3, the movement determination unit 33 determines whether or not each corneal reflection candidate moves over time, based on the characteristic information on each corneal reflection candidate generated by the generation unit 32. For example, the movement determination unit 33 compares a difference between the brightness gradients of each corneal reflection candidate with a threshold. Then, when the difference is not smaller than the threshold, the movement determination unit 33 determines that the corneal reflection candidate moves over time.

As illustrated in FIGs. 6A and 6B, as for the corneal reflection candidate 112, there is no large difference between the brightness gradients (brightness gradient $\theta_{X1}$ ($\theta_{X11}$) and brightness gradient $\theta_{Y1}$ ($\theta_{Y11}$)) in the two or more directions. On the other hand, as illustrated in FIGs. 7A and 7B, as for the corneal reflection candidate 114, there is a large difference between the brightness gradients (brightness gradient $\theta_{X2}$ ($\theta_{X21}$) and brightness gradient $\theta_{Y2}$ ($\theta_{Y21}$)) in the two or more directions.

Moreover, the description is given of the fact that, in the state of FIG. 1B, it is possible to estimate that the sharply outlined corneal reflection candidate 112 is the reflection on the eyeglass surface not in motion and the vaguely outlined corneal reflection candidate 114 is the reflection on the cornea in motion. Thus, in the correspondence relationships among FIG. 1B, FIGs. 6A and 6B, and FIGs. 7A and 7B, it may be determined that the corneal reflection candidate is not in motion when there is no large difference between the brightness gradients (brightness gradient $\theta_{X1}$ and brightness gradient $\theta_{Y1}$) in the two or more directions. On the other hand, it may be determined that the corneal reflection candidate is in motion when there is a large difference between the brightness gradients (brightness gradient $\theta_{X2}$ and brightness gradient $\theta_{Y2}$) in the two or more directions.

Next, when there are two or more regions of the corneal reflection candidates in which a first corneal reflection candidate having a characteristic indicating movement over time coexists with a second corneal reflection candidate having a characteristic indicating no movement over time, the determination unit 34 determines the first corneal reflection candidate as a corneal reflection region representing the corneal reflection. On the other hand, when there are two or more regions of the corneal reflection candidates in which the first corneal reflection candidate having a characteristic indicating movement over time and the second corneal reflection candidate having a characteristic indicating no movement over time do not coexist, the determination unit 34 determines the corneal reflection as unidentifiable. Moreover, when there is only one corneal reflection candidate, the determination unit 34 determines this corneal reflection candidate as the corneal reflection.

For example, the determination unit 34 uses an image in which a corneal reflection candidate having a difference not smaller than a threshold between the brightness gradients (brightness gradient $\theta_{X2}$ and brightness gradient $\theta_{Y2}$) in the two or more directions coexists with a corneal reflection candidate having a difference smaller than the threshold between the brightness gradients (brightness gradient $\theta_{X1}$ and brightness gradient $\theta_{Y1}$) in the two or more directions to detect the corneal reflection candidate with movement as the corneal reflection.

Then, the determination unit 34 inputs the position of the corneal reflection candidate identified as the corneal reflection to the eye-gaze detection unit 37 to be described later. Furthermore, the determination unit 34 stores the position of the corneal reflection candidate identified as the corneal reflection in the storage unit 4 while associating the position with a frame number of the process target image.

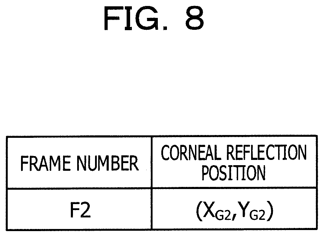

FIG. 8 is a diagram illustrating a data configuration example of the storage unit configured to store corneal reflection information. As the corneal reflection information, the storage unit 4 stores the frame number of the process target image and the corneal reflection position while associating the two with each other. Note that the corneal reflection information is stored to be used for tracking processing by the tracking unit 35 to be described later. FIG. 8 illustrates an example where only latest corneal reflection information is stored. However, corneal reflection information of previous several frames may be stored.

Here, detailed description is given of the determination method by the determination unit 34. For example, when processing is performed on the eye region in FIG. 1B, the movement determination unit 33 makes determination for the corneal reflection candidates 112 and 114 that the corneal reflection candidate 112 has a characteristic without movement and the corneal reflection candidate 114 has a characteristic with movement. In this case, the determination unit 34 determines that the corneal reflection candidate 114 having a characteristic indicating movement over time coexists with the corneal reflection candidate 112 having a characteristic indicating no movement over time, and further determines the corneal reflection candidate 112 as the corneal reflection.

Meanwhile, when processing is performed on the eye region in FIG. 1A, the movement determination unit 33 makes determination for the corneal reflection candidates 102 and 104 that both the corneal reflection candidates 102 and 104 have a characteristic without movement. In this case, since the corneal reflection candidate having a characteristic indicating movement over time and the corneal reflection candidate having a characteristic indicating no movement over time do not coexist (both the corneal reflection candidates 102 and 104 have no movement), the determination unit 34 determines the corneal reflection as unidentifiable.

Furthermore, when processing is performed on the eye region in FIG. 1C, the movement determination unit 33 makes determination for the corneal reflection candidates 122 and 124 that both the corneal reflection candidates 122 and 124 have a characteristic with movement. In this case, since the corneal reflection candidate having a characteristic indicating movement over time and the corneal reflection candidate having a characteristic indicating no movement over time do not coexist (both the corneal reflection candidates 122 and 124 have movement), the determination unit 34 determines the corneal reflection as unidentifiable.

Referring back to FIG. 3, when the determination unit 34 determines the corneal reflection as unidentifiable, the tracking unit 35 tracks the corneal reflection in the process target image, based on the identification result on the previous corneal reflection.

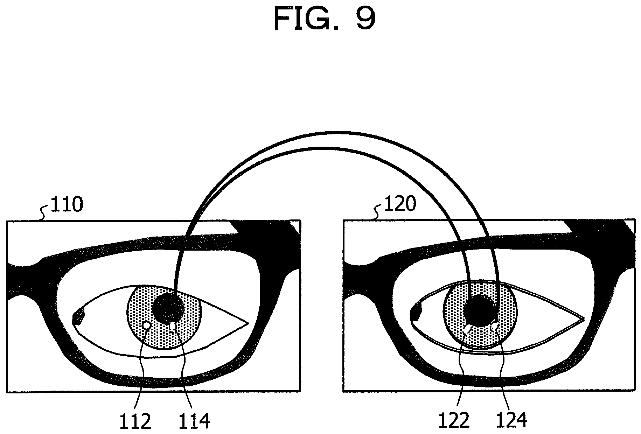

FIG. 9 is a diagram for explaining the tracking processing. A part 110 of the image is equivalent to FIG. 1B. More specifically, it is determined by the determination unit 34 that the corneal reflection candidate 114 is the corneal reflection.

A part 120 of the image is equivalent to FIG. 1C, and is an eye region extracted from an image that is one frame after the part 110 of the image. Hereinafter, the part 120 of the image is referred to as the process target image and the part 110 of the image as the previous image.

The corneal reflection candidate 122 and the corneal reflection candidate 124 in the process target image 120 are both determined to have a characteristic with movement by the movement determination unit 33, and the corneal reflection is determined as unidentifiable by the determination unit 34.

Therefore, the tracking unit 35 uses the corneal reflection 114 in the previous image 110 to determine a corneal reflection candidate closer to the position of the corneal reflection 114 as the corneal reflection. As illustrated in FIG. 9, for example, when the position of the corneal reflection candidate 124 is closer to the position of the corneal reflection 114 than the position of the corneal reflection candidate 122, the corneal reflection candidate 124 is determined as the corneal reflection.

Next, the pupil detection unit 36 detects a pupil. For example, the pupil detection unit 36 detects a region having a predetermined characteristic as the pupil from a near-infrared process target image. Note that a conventional pupil detection technique is adopted for the pupil detection. For example, the pupil detection unit 36 performs matching against templates based on shapes and characteristics of pupils.

The eye-gaze detection unit 37 uses the position of the corneal reflection and the position of the pupil to detect an eye gaze position of the subject. Note that the conventional corneal reflection method is adopted for a method for determining the eye gaze position based on the position of the corneal reflection and the position of the pupil.

The output control unit 38 generates an eye-gaze detection result, and controls the output unit 5 to output the eye-gaze detection result to another device.

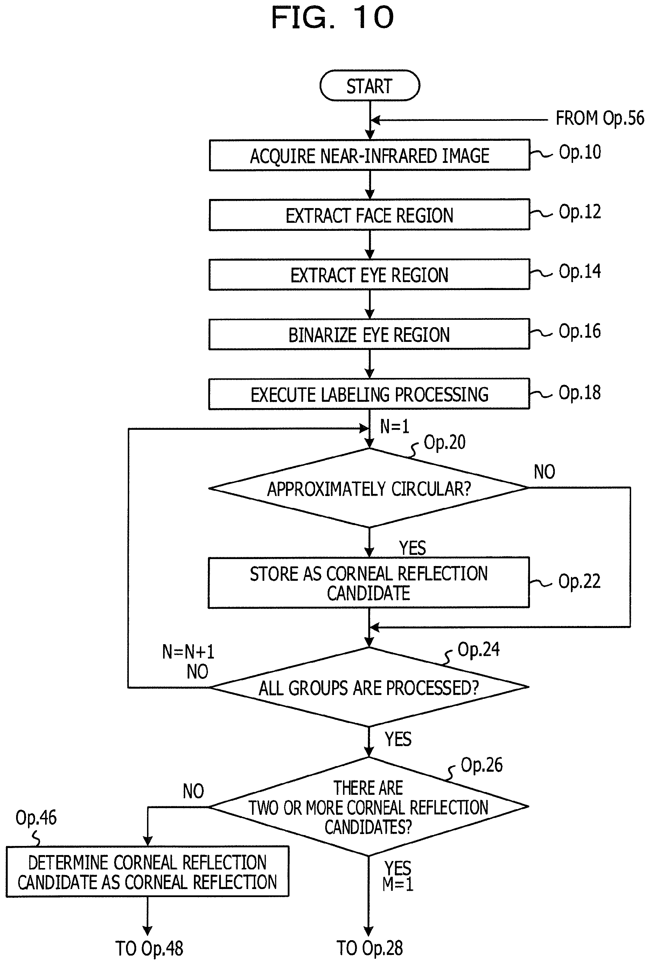

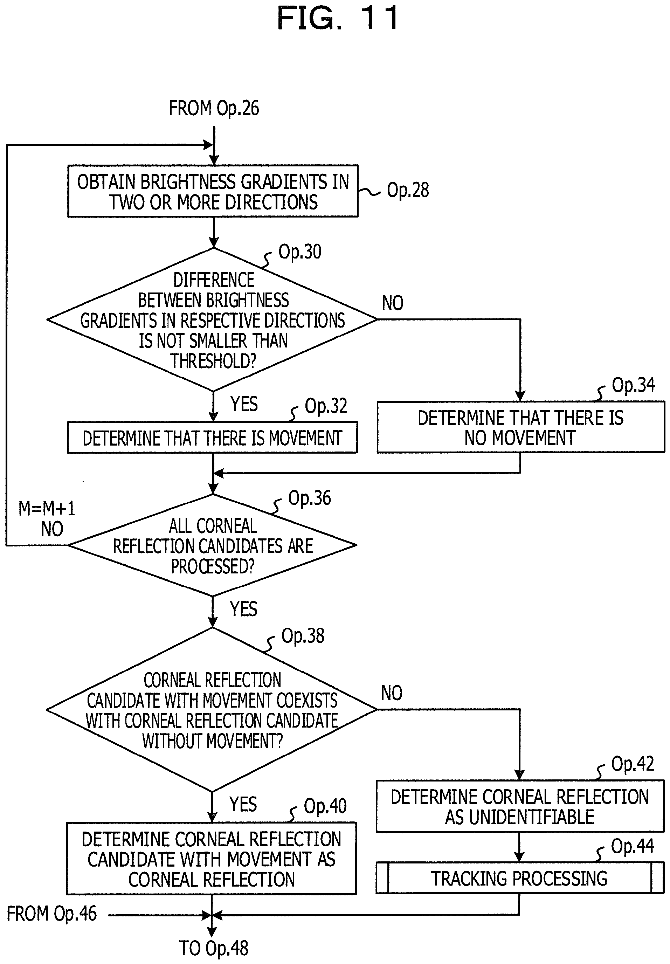

Next, description is given of eye-gaze detection processing including processing of determining the corneal reflection according to this embodiment. FIGs. 10 to 12 are flowcharts illustrating the eye-gaze detection processing.

First, the acquisition unit 2 acquires a near-infrared image from the detection unit 7 (camera) and inputs the near-infrared image to the controller 3 (Op. 10). The extraction unit 31 in the controller 3 extracts a face region from the near-infrared process target image (Op. 12). Furthermore, the extraction unit 31 further extracts an eye region (Op. 14). Next, the extraction unit 31 generates a binarized image by binarizing the eye region (Op. 16). Then, the extraction unit 31 groups regions where a predetermined number of pixels or more with "1" given thereto are assembled, by performing labeling processing on the binarized image (Op. 18). In this event, arbitrary identification information is given to each group. For example, the regions are grouped into Group 1, Group2, ..., and Group n.

With 1 set as an initial value to a counter N indicating identification information of the group (N= 1) and Group 1 as a processing target, the extraction unit 31 determines whether or not the shape of the group is approximately circular (Op. 20). When it is determined that the shape is approximately circular (YES in Op. 20), the extraction unit 31 stores the region determined to be approximately circular as a corneal reflection candidate in the storage unit 4 (Op. 22). To be more specific, coordinates of a barycenter position of the corneal reflection candidate and pixels on the circumference as well as identification information on each corneal reflection candidate are stored in the storage unit 4. For example, when m corneal reflection candidates are extracted, 1 to m integers are given to the respective corneal reflection candidates as the identification information.

On the other hand, when it is determined that the shape is not approximately circular (NO in Op. 20) and after Op. 22, the extraction unit 31 determines whether or not the shape determination is finished for all the groups (Op. 24). When the shape determination is not finished for all the groups (NO in Op. 24), the process target group is changed to next one (N= N+1) and Op. 20 is repeated.

On the other hand, when the shape determination is finished for all the groups (in the case of N= n) (YES in Op. 24), the generation unit 32 determines whether or not there are two or more corneal reflection candidates (Op. 26). When there are not two or more corneal reflection candidates (NO in Op. 26), the generation unit 32 notifies the determination unit 34 to that effect. Then, the determination unit 34 determines one corneal reflection candidate as the corneal reflection, and stores the corneal reflection information in the storage unit 4 (Op. 46).

On the other hand, when there is more than one corneal reflection candidate (YES in Op. 26), the generation unit 32 sets 1 as an initial value to a counter M indicating identification information of the corneal reflection candidate (M= 1), and obtains brightness gradients in the two or more directions of the corneal reflection candidate (Op. 28). For example, a brightness gradient in the X direction and a brightness gradient in the Y direction are obtained using the method described above.

Then, the movement determination unit 33 determines whether or not a difference between the brightness gradients in the respective directions is not smaller than a threshold (Op. 30). When the difference is not smaller than the threshold (YES in Op. 30), the movement determination unit 33 determines that the process target corneal reflection candidate has movement (Op. 32). On the other hand, when the difference is smaller than the threshold (NO in Op. 30), the movement determination unit 33 determines that the process target corneal reflection candidate has no movement (Op. 34).

Next, the movement determination unit 33 determines whether or not the movement determination processing is finished for all the corneal reflection candidates (Op. 36). Note that, when M= m, it is determined that the movement determination processing is finished. When the movement determination processing is not finished for all the corneal reflection candidates (NO in Op. 36), the process target corneal reflection candidate is changed to next one (M= M+1) and Op. 28 is repeated.

On the other hand, when the movement determination processing is finished for all the corneal reflection candidates (YES in Op. 36), the determination unit 34 determines whether or not the corneal reflection candidate with movement coexists with the corneal reflection candidate without movement (Op. 38). When the corneal reflection candidate with movement coexists with the corneal reflection candidate without movement (YES in Op. 38), the determination unit 34 determines the corneal reflection candidate with movement as the corneal reflection, and stores the corneal reflection information in the storage unit 4 (Op. 40).

On the other hand, when the corneal reflection candidate with movement and the corneal reflection candidate without movement do not coexist (NO in Op. 38), the determination unit 34 determines the corneal reflection as unidentifiable and requests the tracking unit 35 for the tracking processing (Op. 42). Upon request of the determination unit 34, the tracking unit 35 executes the tracking processing (Op. 44).

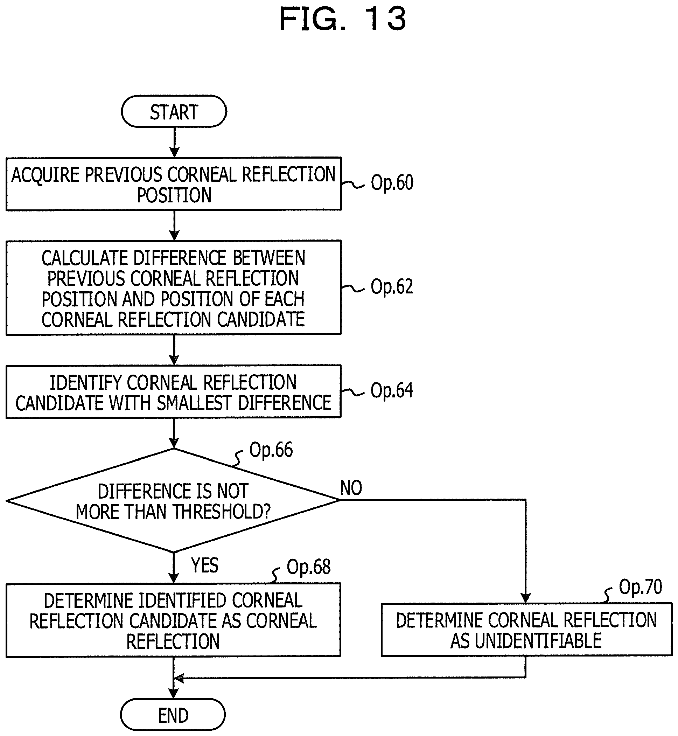

FIG. 13 is a flowchart illustrating the tracking processing. The tracking unit 35 acquires the corneal reflection position of the corneal reflection in the previous image from the storage unit 4 (Op. 60). Note that, here, when information on the corneal reflection in the previous image is not stored in the storage unit 4, the tracking unit 35 terminates the processing, determining that the corneal reflection may not be tracked.

Next, the tracking unit 35 calculates a difference (distance) between the previous corneal reflection position and the position of each of the corneal reflection candidates in the process target image (Op. 62). Then, the tracking unit 35 identifies a corneal reflection candidate having the smallest difference (Op. 64). Furthermore, the tracking unit 35 determines whether or not the difference between the corneal reflection candidates is not larger than a threshold (Op. 66). When the difference is not larger than the threshold (YES in Op. 66), the tracking unit 35 determines the corneal reflection candidate identified in Op. 64 as the corneal reflection, and stores the corneal reflection information in the storage unit 4 (Op. 68). On the other hand, when the difference is larger than the threshold (NO in Op. 66), the tracking unit 35 determines the corneal reflection as unidentifiable (Op. 70). Then, the tracking unit 35 terminates the tracking processing.

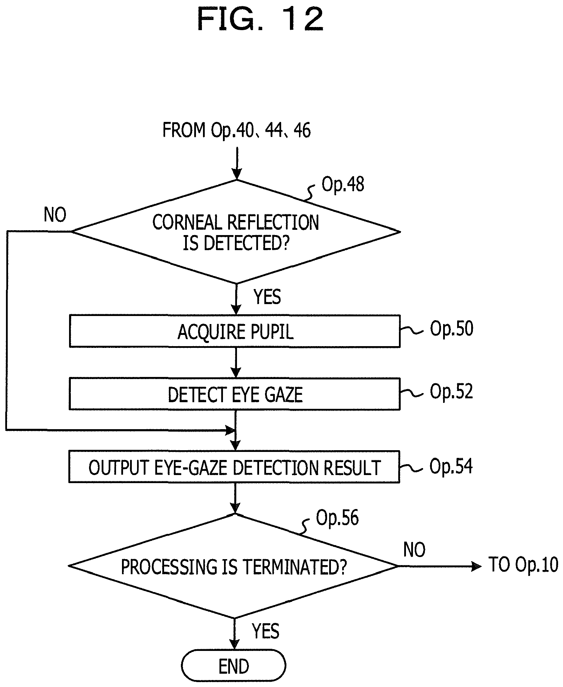

Next, when any of Op. 40, Op. 44, and Op. 46 is finished, Op. 48 in FIG. 12 is executed. More specifically, the determination unit 34 determines whether or not the corneal reflection is detected (Op. 48). When the corneal reflection is detected (YES in Op. 48), the pupil detection unit 36 detects the pupil upon receipt of notification from the determination unit 34 (Op. 50). Furthermore, the eye-gaze detection unit 37 detects the eye gaze of the subject based on the position of the corneal reflection and the position of the pupil (Op. 52).

When no corneal reflection is detected (NO in Op. 48) or when Op. 52 is finished, the output unit 5 outputs the eye-gaze detection result depending on the various processing results to another device under the control of the output control unit 38 (Op. 54). Note that, when no corneal reflection is detected or when no pupil or eye gaze is detected, the information indicating no eye gaze detectable is outputted as the eye-gaze detection result. When the eye gaze is detected, eye gaze information indicating the direction and position of the eye gaze is outputted as the eye-gaze detection result.

Then, the controller 3 determines whether to terminate the eye-gaze detection processing (Op. 56). When terminating the eye-gaze detection processing (YES in Op. 56), the controller 3 terminates the series of eye-gaze detection processing. On the other hand, when not terminating the eye-gaze detection processing (NO in Op. 56), the controller 3 returns to Op. 10 to repeat the series of processing.

Through the above processing, the technique disclosed in this embodiment determines, in an image where a corneal reflection candidate indicating reflection on an object in motion coexists with a corneal reflection candidate indicating reflection on an object not in motion, the former as the corneal reflection, in order to enable eye-gaze detection using the corneal reflection method even if there is eyeglass reflection.

Even when the eyeglass reflection and the corneal reflection coexist, the technique disclosed in this embodiment may distinguish the eyeglass reflection from the corneal reflection by using a characteristic image in which a region of a corneal reflection candidate having a characteristic indicating movement over time coexists with a region of a corneal reflection candidate having a characteristic indicating no movement over time, regardless of the capabilities of the camera.

Moreover, even when the process target image is not the characteristic image, the corneal reflection may be tracked using the detection result of the previous corneal reflection. Therefore, the technique disclosed in this embodiment may detect the corneal reflection even when the eyeglass reflection is generated, thereby improving the accuracy of the eye-gaze detection result using the corneal reflection.

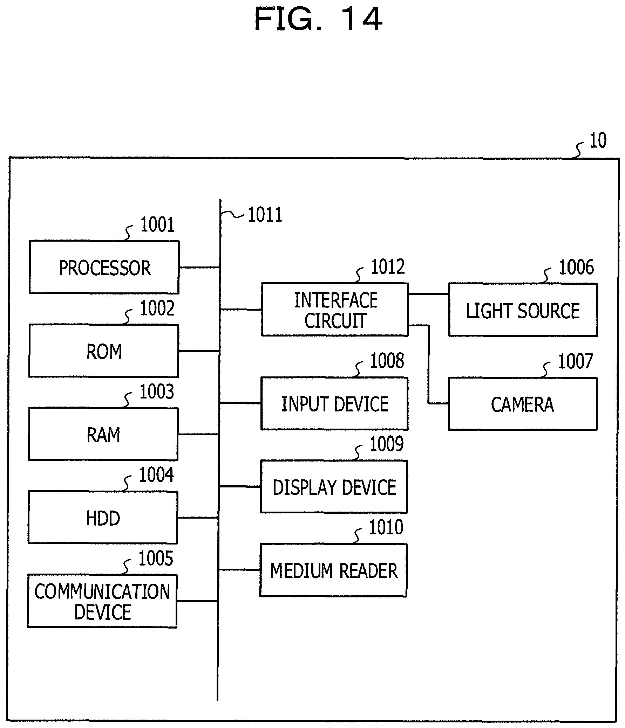

Next, description is given of a hardware configuration example of the eye-gaze detection system including the eye-gaze detection device. FIG. 14 is a diagram illustrating a hardware configuration of the eye-gaze detection system including the eye-gaze detection device. The eye-gaze detection system 10 executes the eye-gaze detection processing according to this embodiment.

The eye-gaze detection device 1 included in the eye-gaze detection system 10 includes, as the hardware configuration, a processor 1001, a Read Only Memory (ROM) 1002, a Random Access Memory (RAM) 1003, a Hard Disk Drive (HDD) 1004, a communication device 1005, an input device 1008, a display device 1009, and a medium reader 1010. Furthermore, the eye-gaze detection system 10 includes an interface circuit 1012, a light source 1006, and a camera 1007, in addition to the hardware configuration of the eye-gaze detection device 1. The processor 1001, the ROM 1002, the RAM 1003, the HDD 1004, the communication device 1005, the input device 1008, the display device 1009, the medium reader 1010, and the interface circuit 1012 are connected to each other through a bus 1011. Accordingly, data may be transmitted and received to and from those described above under the control of the processor 1001.

A program related to the eye-gaze detection processing is recorded in a recording medium that may be read by the eye-gaze detection system 10. The recording medium that may be read by the eye-gaze detection system 10 includes a magnetic recording device, an optical disk, a magneto-optical recording medium, a semiconductor memory, and the like. The magnetic recording device includes an HDD, a flexible disk (FD), a magnetic tape (MT), and the like.

The optical disk includes a Digital Versatile Disc (DVD), a DVD-RAM, a Compact Disc-Read Only Memory (CD-ROM), a Compact Disc-Recordable/ReWritable (CD-R/RW), and the like. The magneto-optical recording medium includes a Magneto-Optical disk (MO), and the like. When distributing a program describing the processing according to the embodiment, it is conceivable to sell a portable recording medium such as a DVD and a CD-ROM recording the program.

The medium reader 1010 in the eye-gaze detection system 10 that executes a program according to this embodiment reads the program from a recording medium recording the program. The processor 1001 stores the read program in the HDD 1004 or in the ROM 1002 or the RAM 1003.

The processor 1001 controls the entire operations of the eye-gaze detection device 1. The processor includes an electronic circuit such as a Central Processing Unit (CPU), for example.

The processor 1001 functions as the acquisition unit 2 and the controller 3 in the eye-gaze detection device 1 by reading the program describing the processing according to this embodiment from the HDD 1004 and executing the program. The communication device 1005 functions as the acquisition unit 2 under the control of the processor 1001. The HDD 1004 stores various information and functions as the storage unit 4 under the control of the processor 1001. The various information may be stored in the ROM 1002 or the RAM 1003 accessible to the processor 1001, as in the case of the program. Furthermore, various information temporarily generated and held through the processing is stored in the RAM 1003, for example.

The input device 1008 receives various inputs. The input device 1008 is a keyboard or a mouse, for example. The display device 1009 displays the various information. The display device 1009 is a display, for example.

As described above, the functional units illustrated in FIGs. 2 and 3 are realized by the hardware including the processor 1001 and the memory (HDD 1002, ROM 1002, and RAM 1003). Then, the eye-gaze detection processing illustrated in FIGs. 10 to 12 is executed by the processor 1001 reading and executing the program stored in the memory.

Moreover, the eye-gaze detection processing according to this embodiment may be executed on cloud. In this case, the light source 6 and the detection unit 7 are disposed in a space where the subject is present. Then, upon receipt of the detection result from the detection unit 7, the eye-gaze detection device 1 (one or more servers) executes the eye-gaze detection processing illustrated in FIGs. 10 to 12.

REFERENCE SIGNS LIST

| 10 | eye-gaze detection system |
|---|---|
| 1 | eye-gaze detection device |
| 2 | acquisition unit |
| 3 | controller |
| 4 | storage unit |
| 5 | output unit |
| 6 | light source |
| 7 | detection unit |
| 1001 | processor |
| 1002 | ROM |
| 1003 | RAM |
| 1004 | HDD |
| 1005 | communication device |
| 1006 | light source |
| 1007 | camera |
| 1008 | input device |
| 1009 | display device |
| 1010 | medium reader |
| 1011 | bus |
| 1012 | interface circuit |

**Claims**

1. A detection system for detecting corneal reflection to be used to detect an eye gaze of a subject, comprising:

   a detection unit configured to detect reflected light from a subject side irradiated with light from a light source and generate an image based on a detection result of the reflected light;
   an extraction unit configured to extract a region from the image, the region having an intensity of the reflected light which is equal to or larger than a specific value; and
   a determination unit configured to, when there are two or more regions, as a extraction result of the region, in which a first region having a characteristic indicating movement over time coexist with a second region having another characteristic indicating no movement over time, determine the first region as the corneal reflection.

2. The detection system according to claim 1, further comprising:

   a pupil detection unit configured to extract a pupil of the subject from the image; and
   an eye-gaze detection unit configured to detect the eye gaze of the subject based on the pupil and the corneal reflection.

3. The detection system according to claim 1 or 2, further comprising:

   a generation unit configured to generate, when there are the two or more regions, characteristic information for each of the regions, the characteristic information indicating a characteristic that varies depending on presence or absence of the movement; and
   a movement determination unit configured to determine whether there is the movement for each of the regions, based on the characteristic information, and
   wherein the determination unit determines the corneal reflection based on the determination result obtained by the movement determination unit.

4. The detection system according to claim 3, wherein the characteristic information is brightness gradients in at least two directions in the region.

5. The detection system according to claim 4, wherein the movement determination unit obtains a difference between the brightness gradients in the at least two directions for each of the regions, and determines that the first region where the difference is not smaller than a threshold has the characteristic indicating movement and that the second region where the difference is smaller than the threshold has the another characteristic indicating no movement.

6. The detection system according to claim 3, wherein the characteristic information is an index for evaluating ellipticity of a shape of the region.

7. The detection system according to any one of claims 1 to 6, wherein
   the region has an approximately circular shape.

8. The detection system according to any one of claims 1 to 7, wherein
   the intensity of the reflected light is a brightness value of each pixel in the image.

9. A detection method for detecting corneal reflection to be used to detect an eye gaze of a subject, comprising:

   acquiring an image from a detection unit which detects reflected light from a subject side irradiated with light from a light source and generate an image based on a detection result of the reflected light;

extracting a region from the image, the region having an intensity of the reflected light which is equal to or larger than a specific value;

when there are two or more regions, as a extraction result of the region, in which a first region having a characteristic indicating movement over time coexist with a second region having another characteristic indicating no movement over time, determining the first region as the corneal reflection; and

detecting the eye gaze of the subject based on the corneal reflection.

**10.** The detection method according to claim 9, further comprising:

extracting a pupil of the subject from the image; and

detecting the eye gaze of the subject based on the pupil and the corneal reflection.

**11.** The detection method according to claim 9 or 10, further comprising:

generating, when there are the two or more regions, characteristic information for each of the regions, the characteristic information indicating a characteristic that varies depending on presence or absence of the movement;

determining whether there is the movement for each of the regions, based on the characteristic information; and

determining the corneal reflection based on a determination result regarding whether there is the movement for each of the regions.

**12.** The detection method according to claim 11, wherein the characteristic information is brightness gradients in at least two directions in the region.

**13.** The detection method according to claim 12, further comprising:

obtaining a difference between the brightness gradients in the at least two directions for each of the regions; and

determining that the first region where the difference is not smaller than a threshold has the characteristic indicating movement and that the second region where the difference is smaller than the threshold has the another characteristic indicating no movement.

**14.** The detection method according to claim 11, wherein the characteristic information is an index for evaluating ellipticity of a shape of the region.

**15.** A detection program which, when executed on a computer, causes the computer to carry out the detection method according to any one of claims 9 to 14.

FIG. 1A

FIG. 1B

FIG. 1C

100

102    104

102

110

112    114

112    114

120

122    124

122

# FIG. 2

# FIG. 3

3

| 31 |
|---|
| EXTRACTION UNIT |

| 32 |
|---|
| GENERATION UNIT |

| 33 |
|---|
| MOVEMENT DETERMINATION UNIT |

| 34 |
|---|
| DETERMINATION UNIT |

| 35 |
|---|
| TRACKING UNIT |

| 36 |
|---|
| PUPIL DETECTION UNIT |

| 37 |
|---|
| EYE-GAZE DETECTION UNIT |

| 38 |
|---|
| OUTPUT CONTROL UNIT |

# FIG. 4

| IDENTIFICATION INFORMATION | BARYCENTER POSITION | CIRCUMFERENTIAL PIXEL | CIRCUMFERENTIAL PIXEL | CIRCUMFERENTIAL PIXEL | CIRCUMFERENTIAL PIXEL |
|---|---|---|---|---|---|
| 1 | $(X_{G1}, Y_{G1})$ | $(X_{11}, Y_{11})$ | $(X_{12}, Y_{12})$ | $(X_{13}, Y_{13})$ | $(X_{14}, Y_{14})$ |
| 2 | $(X_{G2}, Y_{G2})$ | $(X_{21}, Y_{21})$ | $(X_{22}, Y_{22})$ | $(X_{23}, Y_{23})$ | $(X_{24}, Y_{24})$ |
| . . . | . . . | . . . | . . . | . . . | . . . |

# FIG. 5A

# FIG. 5B

112

$(X_{12}, Y_{12})$

$(X_{11}, Y_{11})$ $(X_{G1}, Y_{G1})$ $(X_{13}, Y_{13})$

$(X_{14}, Y_{14})$

114

$(X_{22}, Y_{22})$

$(X_{21}, Y_{21})$ $(X_{G2}, Y_{G2})$ $(X_{23}, Y_{23})$

$(X_{24}, Y_{24})$

# FIG. 6A

BRIGHTNESS

# FIG. 6B

BRIGHTNESS

# FIG. 7A

BRIGHTNESS

# FIG. 7B

BRIGHTNESS

# FIG. 8

| FRAME NUMBER | CORNEAL REFLECTION POSITION |
|:---:|:---:|
| F2 | $(X_{G2}, Y_{G2})$ |

# FIG. 9

# FIG. 10

START

FROM Op.56

ACQUIRE NEAR-INFRARED IMAGE — Op.10

EXTRACT FACE REGION — Op.12

EXTRACT EYE REGION — Op.14

BINARIZE EYE REGION — Op.16

EXECUTE LABELING PROCESSING — Op.18

$N=1$

Op.20

APPROXIMATELY CIRCULAR? — NO

YES

STORE AS CORNEAL REFLECTION CANDIDATE — Op.22

$N=N+1$
NO

Op.24

ALL GROUPS ARE PROCESSED?

YES

Op.26

THERE ARE TWO OR MORE CORNEAL REFLECTION CANDIDATES? — NO

YES
$M=1$

Op.46

DETERMINE CORNEAL REFLECTION CANDIDATE AS CORNEAL REFLECTION

TO Op.48

TO Op.28

# FIG. 11

FROM Op.26

OBTAIN BRIGHTNESS GRADIENTS IN TWO OR MORE DIRECTIONS — Op.28

Op.30

DIFFERENCE BETWEEN BRIGHTNESS GRADIENTS IN RESPECTIVE DIRECTIONS IS NOT SMALLER THAN THRESHOLD?

NO

Op.34

DETERMINE THAT THERE IS NO MOVEMENT

YES — Op.32

DETERMINE THAT THERE IS MOVEMENT

M=M+1
NO

Op.36

ALL CORNEAL REFLECTION CANDIDATES ARE PROCESSED?

YES

Op.38

CORNEAL REFLECTION CANDIDATE WITH MOVEMENT COEXISTS WITH CORNEAL REFLECTION CANDIDATE WITHOUT MOVEMENT?

NO

Op.42

DETERMINE CORNEAL REFLECTION AS UNIDENTIFIABLE

YES — Op.40

DETERMINE CORNEAL REFLECTION CANDIDATE WITH MOVEMENT AS CORNEAL REFLECTION

Op.44

TRACKING PROCESSING

FROM Op.46

TO Op.48

23

# FIG. 12

FROM Op.40、44、46

Op.48

NO ← CORNEAL REFLECTION IS DETECTED?

YES

ACQUIRE PUPIL — Op.50

DETECT EYE GAZE — Op.52

OUTPUT EYE-GAZE DETECTION RESULT — Op.54

Op.56

PROCESSING IS TERMINATED? — NO → TO Op.10

YES

END

# FIG. 13

START

ACQUIRE PREVIOUS CORNEAL REFLECTION POSITION — Op.60

CALCULATE DIFFERENCE BETWEEN PREVIOUS CORNEAL REFLECTION POSITION AND POSITION OF EACH CORNEAL REFLECTION CANDIDATE — Op.62

IDENTIFY CORNEAL REFLECTION CANDIDATE WITH SMALLEST DIFFERENCE — Op.64

Op.66

DIFFERENCE IS NOT MORE THAN THRESHOLD?

NO

YES

Op.68

DETERMINE IDENTIFIED CORNEAL REFLECTION CANDIDATE AS CORNEAL REFLECTION

Op.70

DETERMINE CORNEAL REFLECTION AS UNIDENTIFIABLE

END

# FIG. 14

**EP 3 207 861 A2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006167256 A

- JP 2012239550 A

**Non-patent literature cited in the description**

- **TAKEHIKO OHNO et al.** *the research report of Information Processing Society of Japan, 2001-HI-93,* 2001, 47-54
- **WILHELM BURGER.** *Digital Image Processing,* 224-225

- **KENICHIRO MURAMOTO et al.** Analysis of Snowflake Shape by a Region and Contour Approach. *The Journal of the Institute of Electronics, Information and Communication Engineers,* May 1993, vol. J76-D-II (5), 949-958